# EUROPEAN PATENT APPLICATION

(11) **EP 1 407 747 A1**
(43) Date of publication of application: **14.04.2004**
(21) Application number: 03103732.8
(22) Date of filing: 08.10.2003
(51) Int. Cl.: A61F 13/15

(54) **Absorbent article**

(30) Priority: 09.10.2002 JP 2002295916
(71) Applicant: Zuiko Corporation, Osaka 566-0045 (JP)
(72) Inventor: ICHIURA, Yuzo, 567-0845, Ibaraki (JP); NAKAKADO, Masaki, 573-0065, Hirakata (JP); KURATA, Syuhei, 6111-0002, Uji (JP); MURATA, Natsumi, 537-0001, Osaka (JP)
(74) Representative: Suckling, Andrew Michael

(57) **Abstract**

An absorbent article, comprising a main part (2), the main part (2) including a liquid-permeable surface sheet (21), a liquid-impermeable anti leak sheet (22), and a liquid-holding absorbent (23) provided between the surface sheet (21) and the anti leak sheet. The main part (2) is folded into a plurality of layers, and portions of the folded main part (2) that are overlapping with one another along opposite side edges of the main part (2) are bonded together.

## Description

### BACKGROUND OF THE INVENTION

### FIELD OF THE INVENTION:

The present invention relates to an absorbent article that is put on the inside of the user's underwear in a narrow crotch area, such as a sanitary napkin, an incontinence pad, a sanitary pad for vaginal discharges, or the like.

### DESCRIPTION OF THE RELATED ART:

Typically, an absorbent article such as a sanitary napkin at least includes an elongate main part, including a liquid-permeable surface sheet, a liquid-impermeable anti leak sheet, and a liquid-holding absorbent provided between the surface sheet and the anti leak sheet. The absorbent article is placed on a packaging sheet that is large enough to extend beyond the front, back, first and second edges of the main part, with the anti leak sheet side of the absorbent article facing the packaging sheet. The absorbent article is folded in three in the longitudinal direction. Specifically, the front portion of the main part is folded, together with the front portion of the packaging sheet, onto the middle portion of the main part, and the rear portion of the main part is folded, together with the rear portion of the packaging sheet, onto the already-folded front portion of the packaging sheet, thereby obtaining an individually-packaged, three-fold absorbent article (see, for example, Japanese Laid-Open Patent Publication Nos. 9-10257 and 9-327478).

Since the absorbent article is individually packaged with the packaging sheet, the folded absorbent article (particularly the surface sheet thereof , which is to be in contact with the user' s skin) can be kept unexposed until use. However, before using the absorbent article, the user needs to open the packaging sheet, and the packaging sheet needs to be disposed of as a waste.

Thus, the provision of the packaging sheet for individually packaging the absorbentarticle notonly consumesnatural resources but also imposes an additional cost for the disposal thereof.

### SUMMARY OF THE INVENTION

It is an object of the present invention to provide an absorbent article that is kept folded while keeping its surface sheet unexposed until use, without using a packaging sheet.

An absorbent article of the present invention at least includes an elongate main part, the main part including a liquid-permeable surface sheet, a liquid-impermeable anti leak sheet, and a liquid-holding absorbent provided between the surface sheet and the anti leak sheet, wherein the main part is folded into a plurality of layers, and portions of the surface sheet and the anti leak sheet of the folded main part that are overlapping with one another along opposite side edges of the main part are bonded together.

Thus, the main part is folded into a plurality of layers, and portions of the folded main part that are overlapping with one another along the opposite side edges of the main part are bonded together, whereby the absorbent article can be kept folded while keeping the surface sheet, which is to be in contact with the user's skin, unexposed.

As a result, it is no longer necessary to provide a packaging sheet for individually packaging the absorbent article, thereby conserving natural resources and saving the cost for the disposal thereof.

In the present invention, a wing portion may extend from each side edge of the surface sheet and the anti leak sheet of the main part. In such a case, as the wing portions are folded back onto the outer surface of the user's underwear, a portion of each side edge portion of the main part, which is used as a bonding portion, is also folded back onto the outer surface of the user's underwear. Thus, it is possible to prevent a bonding portion from contacting and irritating the user's skin.

In the present invention, each wing portion may be folded onto the surface sheet with an adhesive being applied on an outer surface thereof, and a release liner may be attached to the wing portions so as to extend between the adhesive application areas of the wing portions. Thus, the wing portions can be kept folded onto the surface sheet. As the release liner is peeled off and the wing portions are folded back onto the outer surface of the user's underwear, the wing portions can be attached to the outer surface of the user' s underwear via the adhesive application areas. Thus, the absorbent article can be fixed on the user's underwear. In the present invention, an adhesive may be applied to at least a portion of the anti leak sheet, with a release liner being attached to the adhesive application area of the anti leak sheet, while the main part is folded. Then, when using the absorbent article, the absorbent article can be fixed on the user's underwear.

### BRIEF DESCRIPTION OF THE DRAWINGS

FIG. **1** is a perspective view illustrating a sanitary napkin as an absorbent article of the present invention according to one embodiment of the present invention.
FIG. **2** is a cross-sectional view illustrating the sanitary napkin of FIG. **1** taken along line X-X in FIG. **1**.
FIG. **3** is a perspective view illustrating the sanitary napkin of FIG. **1** after unfolding the sanitary napkin by peeling off the bonded portions.
FIG. **4** is a perspective view illustrating the sanitary napkin of FIG. **1** immediately before it is put on the user' s underwear.
FIG. **5A** is a plan view illustrating a sanitary napkin as an absorbent article of the present invention according to an alternative embodiment of the present invention, FIG. **5B** is a plan view illustrating the sanitary napkin of FIG. **5A** after unfolding the sanitary napkin by peeling off the bonded portions, and FIG. **5C** is a cross-sectional view taken along line Y-Y in FIG. **5A.**
FIG. **6A** is a plan view illustrating a sanitary napkin as an absorbent article of the present invention according to another alternative embodiment of the present invention, FIG. **6B** is a plan view illustrating the sanitary napkin of FIG. **6A** after unfolding the sanitary napkin by peeling off the bonded portions, and FIG. **6C** is a cross-sectional view taken along line Y-Y in FIG. **6A.**

### DESCRIPTION OF THE PREFERRED EMBODIMENTS

Preferred embodiments of the present invention will now be described with reference to the drawings.

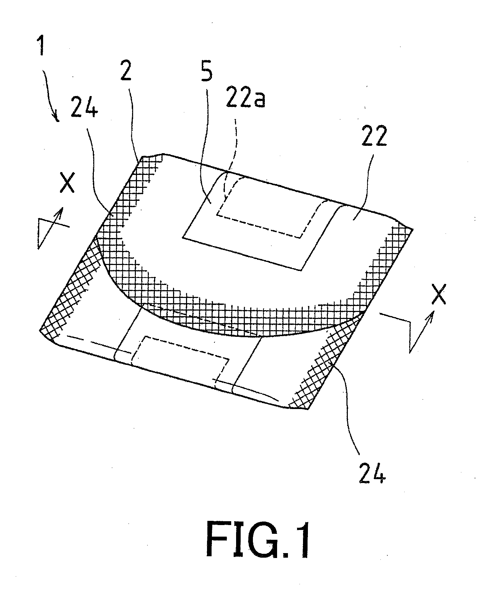

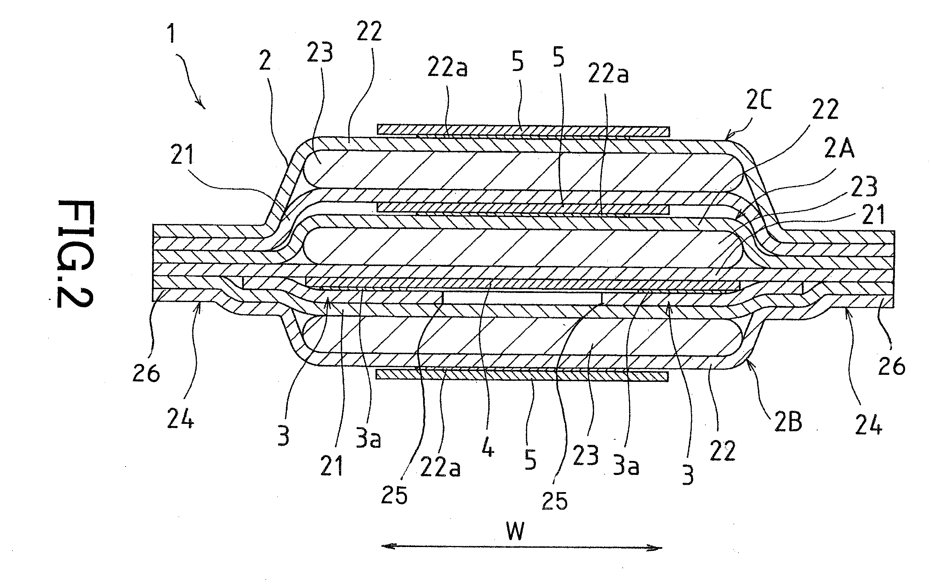

FIG. **1** and FIG. **2** illustrate a sanitary napkin **1** as an absorbent article of the present invention according to one embodiment of the present invention.

The sanitary napkin **1** includes an elongate main part **2**, and a pair of wing portions 3 having a generally trapezoidal shape that are integral respectively with first and second side edge portions **24** of the main part **2** in a middle portion **2B,** which is located generally in the middle of the main part **2**. The first side edge opposes to the second side edge. The main part **2** includes a liquid-permeable surface sheet **21,** a liquid-impermeable anti leak sheet **22 ,** and a liquid-holding absorbent **23** provided between the surface sheet **21** and the anti leak sheet **22** . The wing portions **3** extend respectively from the first and second side edge portions **24** of the main part **2** (more specifically, from the first and second side edge portions of the surface sheet **21**).

Each wing portion **3** is normally folded onto the surface sheet **21,** and includes an adhesive application area **3a** where an adhesive is applied on the surface of the wing portion **3 .** Moreover, a release liner 4 is attached to the adhesive application areas 3a of the first and second wing portions **3** so as to extend between the adhesive application areas **3a,** whereby the free end **25** of each wing portion **3** is held in contact with the surface sheet **21** so as not to extend in the width direction W beyond the first or second side edge portion **24** of the main part **2**.

Moreover, the anti leak sheet **22** includes an adhesive application area **22a** where an adhesive is applied on the surface of the anti leak sheet **22** in the longitudinal direction L, and a release liner **5** is attached to the adhesive application area **22a.**

Note that the wing portions **3** may be connected to the anti leak sheet **22**, instead of connecting them to the surface sheet **21.** For example, in a case where portions **26** of the anti leak sheet **22** are not covered by the surface sheet **21** at the first and second side edge portions **24** of the main part **2**, the wing portions **3** may be connected to the portions **26**.

The sanitary napkin **1** is folded in three with the wing portions **3** being folded onto the surface sheet **21** of the main part **2**. Specifically, a front portion **2A** of the main part **2** is folded onto the middle portion **2B**, with the surface sheet **21** of the front portion **2A** being in contact with the surface sheet **21** of the middle portion **2B,** and then a rear portion **2C** is folded onto the already-folded front portion **2A,** with the surface sheet **21** of the rear portion **2C** being in contact with the anti leak sheet **22** of the front portion **2A,** thereby obtaining a three-fold structure. Then, portions of the surface sheet **21** and the anti leak sheet **22** of the main part **2** that are overlapping with one another along the first and second side edge portions **24** of the sanitary napkin **1**, which has been folded into a three-fold structure, are bonded togetherbyusingthermalfusionbonding, ultrasonic fusion bonding, an adhesive, or the like. Thus, the surface sheet **21**, which is to be in contact with the user's skin, is kept unexposed.

The bonding strength between the surface sheet **21** of the front portion **2A** and the surface sheet **21** of the middle portion **2B**, when the napkin **1** is folded, is set to be smaller than that between the wing portions **3** and the surface sheet **21**. Moreover, the bonding strength between the surface sheet **21** of the rear portion **2C** and the anti leak sheet **22** of the front portion **2A,** when the napkin **1** is folded, is set to be smaller than that between the wing portions **3** and the surface sheet **21.** Furthermore, in a case where the surface sheet **21** of the front portion **2A** and the wing portions **3** are bonded to each other when the napkin **1** is folded, the bonding strength therebetween is set to be smaller than that between the wing portions **3** and the surface sheet **21** (or the anti leak sheer **22**) of the middle portion **2B.**

As a result, the sanitary napkin **1** is folded into a three-fold structure and is kept in the three-fold structure by bonding along the first and second side edge portions **24,** so that the surface sheet **21**, which is to be in contact with the user's skin, is kept unexposed. Therefore, it is no longer necessary to provide a packaging sheet, thereby conserving natural resources and saving the cost for the disposal thereof.

Next, how to use the sanitary napkin 1 as described above will be described.

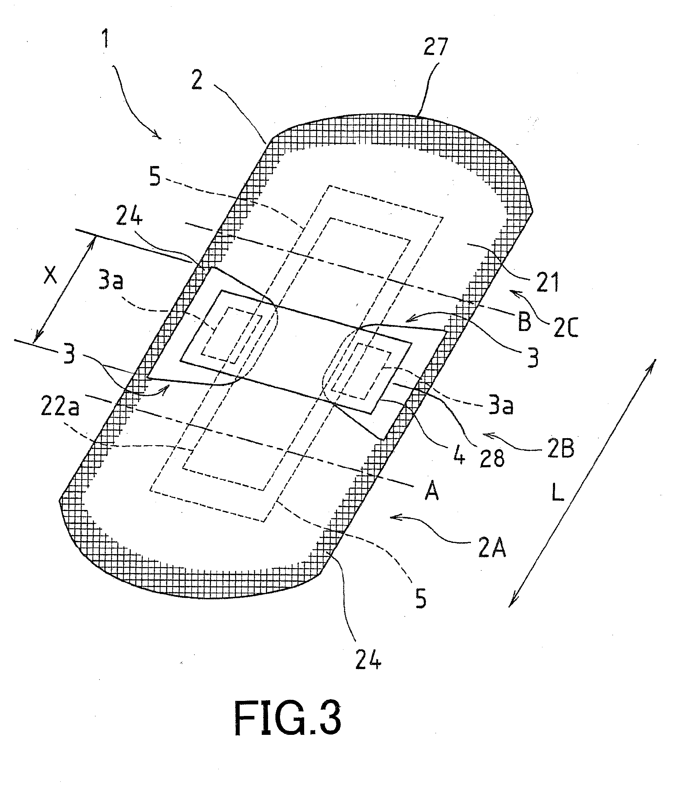

First, as illustrated in Fig. **3**, the user takes the tip **27** of the rear portion **2C** of the main part **2** of the three-fold sanitary napkin **1**, and unfolds the rear portion **2C**, thereby peeling off the first and second side edge portions of the surface sheet **21** and the anti leak sheet **22** of the rear portion **2C** from those of the front portion **2A** so that the rear portion **2C** forms a continuous flat surface with the middle portion **2B.** Similarly, the user takes the tip of the front portion **2A** of the main part **2**, and unfolds front portion **2A**, thereby peeling off the side edge portions **24** of the surface sheet **21** and the anti leak sheet **22** of the front portion **2A** from those of the middle portion **2B** so that the front portion **2A** forms a continuous flat surface with the middle portion **2B** (see FIG. **3**).

Then, the release liner 5 attached to the anti leak sheet **22** is peeled off from the adhesive application area **22a,** and the adhesive application area **22a** is attached to the crotch portion of the inner surface of the user's underwear. Then, the release liner **4** is peeled off from the adhesive application areas **3a** of the wing portions **3**, and the wing portions **3** are folded back around the crotch portion of the underwear to be attached to the outer surface thereof. Thus, the sanitary napkin 1 can be fixed on the underwear.

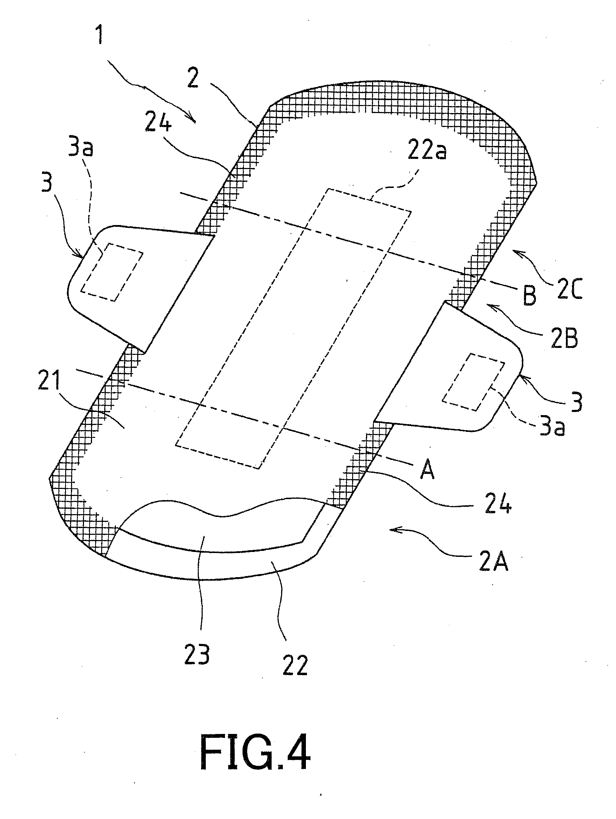

Fig. **4** illustrates a plan view of the sanitary napkin **1** just prior to being applied to the user's underwear. As the wing portions **3** are folded back outwardly, a portion of each side edge portion of the surface sheet **21** and the anti leak sheet **22** of the main part **2**, i.e., each side edge portion of the middle portion **2B,** which is used as a bonding portion, is also folded back onto the outer surface of the user's underwear, together with the wing portions **3**, thus preventing a bonding portion from contacting and irritating the user's skin.

While the present embodiment is directed to the sanitary napkin **1** including the wing portions **3**, the present invention is not limited to the sanitary napkin **1**, and the wing portions **3** may be optional under certain circumstances.

While the wing portions **3** are provided in the middle portion **2B** of the main part **2** in the present embodiment, the position where the wing portions **3** are provided is not limited to any particular position, and they may alternatively be provided in the front portion **2A** or the rear portion **2C**, for example, as long as the sanitary napkin **1** can be fixed on the user's underwear.

While the sanitary napkin **1** is folded into a three-fold structure in the present embodiment, it may alternatively be folded in two or four, for example.

While the wing portions **3** are provided in the middle portion **2B** of the main part **2** in the present embodiment so that when the wing portions **3** are folded back, the side edge portions **24** of the middle portion **2B**, which are used as bonding portions, are folded back onto the outer surface of the user's underwear, it may be preferred that the side edge portions **24** of the main part **2** are entirely folded back onto the outer surface of the underwear by, for example, increasing the width X of the foot portion **28** of the wing portions **3**.

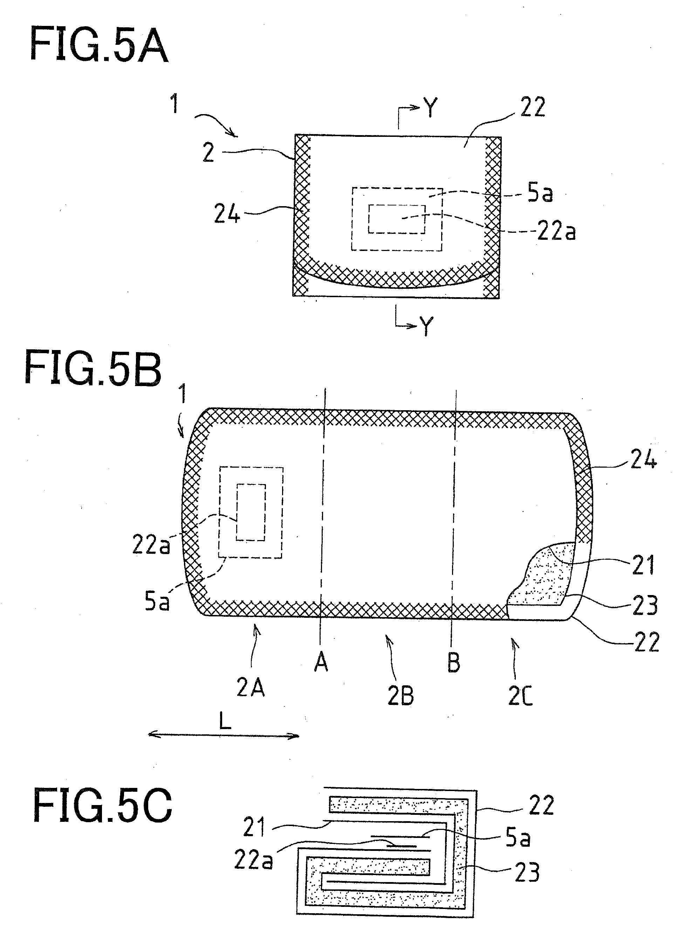

While an adhesive is applied on the surface of the anti leak sheet **22** to provide the adhesive application area **22a** in the present embodiment, the adhesive application area **22a** may be optional. An alternative embodiment of the present invention will now be described with reference to FIG. **5A** to FIG. **5C.** As illustrated in FIG. **5A,** the sanitary napkin **1** of the present embodiment includes the elongate main part **2**, including the liquid-permeable surface sheet **21,** the liquid-impermeable anti leak sheet **22**, and the liquid-holding absorbent **23** provided between the surface sheet **21** and the anti leak sheet **22.** Moreover, the anti leak sheet **22** includes the adhesive application area **22a,** where an adhesive is applied, near one end of the anti leak sheet **22** in the longitudinal direction L , and a release liner **5a** is attached to the adhesive application area **22a.** The main part **2** is folded in three so that the release liner **5a** is kept unexposed. Specifically, referring to FIG. **5B,** an end portion **2A** of the main part **2** is folded along line A onto a middle portion **2B** so that the surface sheet of the end portion **2A** faces the surface sheet of the middle portion **2B,** and an end portion **2C** is folded along line B, thereby forming a three-fold sanitary napkin. As a result, the main part **2** is folded so that the adhesive application area **22a** and the release liner **5a** are kept unexposed, with the surface sheet of the end portion **2C** facing the release liner **5a,** as illustrated in FIG. **5C.** Then, portions of the surface sheet **21** and the anti leak sheet **22** of the main part 2 that are overlapping with one another along the first and second side edge portions **24** of the sanitary napkin **1**, which has been folded into a three-fold structure, are bonded together by using thermal fusion bonding, ultrasonic fusion bonding, an adhesive, or the like. Thus, the surface sheet **21,** which is to be in contact with the user's skin, is kept unexposed. While the adhesive application area **22a** is provided near one of the main part **2** in the present embodiment, the present invention is not limited to this as long as it is provided near at least one end of the anti leak sheet **22.** For example, adhesive application areas may be provided near both end portions **2A** and **2C** of the main part **2**. Moreover, the release liner may be partially protruding out of the main part **2** as it is folded. Furthermore, the main part **2** may alternatively include wing portions as described above.

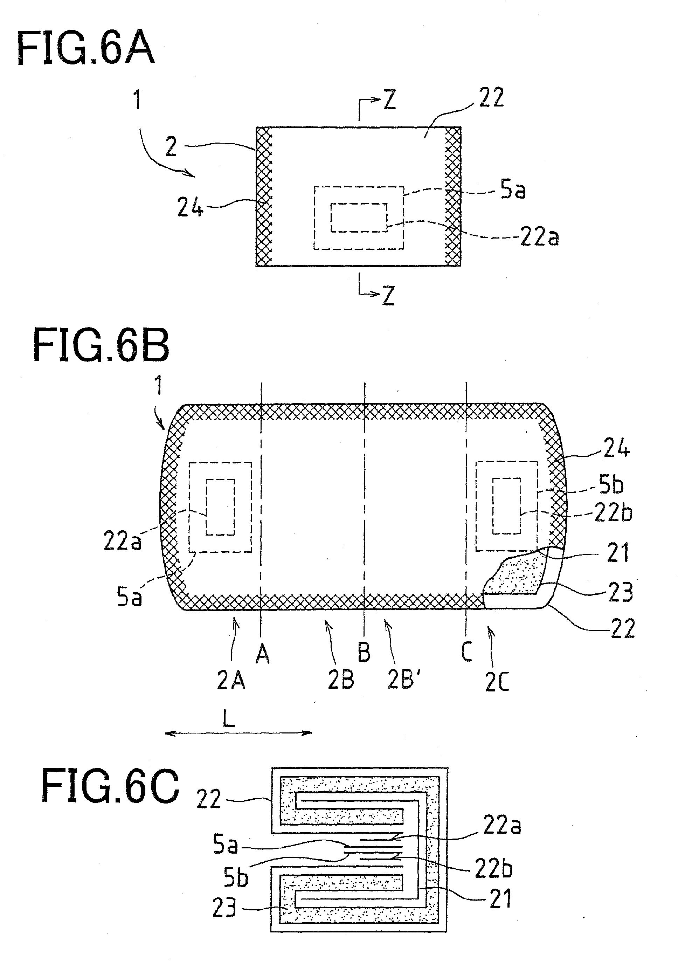

Next, another alternative embodiment of the present invention will be described with reference to FIG. **6A** to FIG. **6C.** As illustrated in FIG. **6A** to FIG. **6C,** the sanitary napkin **1** of the present embodiment includes the elongate main part **2** , including the liquid-permeable surface sheet **21,** the liquid-impermeable anti leak sheet **22**, and the liquid-holding absorbent **23** provided between the surface sheet **21** and the anti leak sheet **22.** Moreover, the anti leak sheet **22** includes adhesive application areas **22a** and **22b,** where an adhesive is applied, near opposite ends of the anti leak sheet **22** in the longitudinal direction L, and release liners 5a and 5b are attached to the adhesive application areas 22a and **22b,** respectively. The sanitary napkin **1** is folded in four so that the release liners **5a** and **5b** are kept unexposed. Specifically, referring to FIG. **6B,** an end portion **2A** is folded along line A onto a middle portion **2B** so that the surface sheet **21** of the end portion **2A** faces the surface sheet **21** of the middle portion **2B.** Similarly, an end portion **2C** is folded along line **C** onto a middle portion **2B'** so that the surface sheet **21** of the end portion **2C** faces the surface sheet **21** of the middle portion **2B'.** The thus-folded main part **2** is further folded along line B, thereby forming a four-fold sanitary napkin. As a result, the main part **2** is folded so that the adhesive application areas **22a** and **22b** and the release liners **5a** and **5b** are kept unexposed, as illustrated in FIG. **6C.** Then, portions of the surface sheet **21** and the anti leak sheet **22** of the main part **2** that are overlapping with one another along the first and second side edge portions **24** of the sanitary napkin **1**, which has been folded into a four-fold structure, are bonded together by using thermal fusion bonding, ultrasonic fusion bonding, an adhesive, or the like. Thus, the surface sheet **21**, which is to be in contact with the user' s skin, is kept unexposed.

While the adhesive application areas **22a** and **22b** are provided near opposite ends of the main part **2** in the present embodiment, the present invention is not limited to this as long as it is provided near at least one of the anti leak sheet **22.** For example, an adhesive application area may be provided near only one end , such as end portion **2A** of the main part **2**. Moreover, the release liners may be partially protruding out of the main part **2** as it is folded. In such a case, it is preferred that the protruding portions of the release liners are folded outwardly so that they do not add to the bulkiness of the folded sanitary napkin **1**. Furthermore, the main part **2** may alternatively include wing portions as described above. Moreover, a single release liner may alternatively be provided, while being folded, between the adhesive application areas **22a** and **22b.** In such a case, it is preferred that at least one of the adhesive application areas **22a** and **22b** is formed in, for example, astripedpattern, acrosspattern, a dotted pattern, or the like, so that the end portion **2A** or **2C** is not entirely an adhesive application area, whereby the air trapped inside as the sanitary napkin **1** is folded can escape out of the folded sanitary napkin **1**. Moreover, in such a case, it is preferred that the release liners are partially protruding out of the main part **2** so that the user can take the protruding portion to unfold the sanitary napkin **1** for use. It is preferred that the protruding portions of the release liners are folded outwardly so that they do not add to the bulkiness of the folded sanitary napkin **1**.

Thus, according to the present invention, it is possible to keep an absorbent article such as a sanitary napkin folded until use without using a packaging sheet. Therefore, it is no longer necessary to provide a packaging sheet for individually packaging the absorbent article, thereby conserving natural resources and saving the cost for the disposal thereof.

## Claims

1. An absorbent article, comprising a main part (2), the main part (2) including a liquid-permeable surface sheet (21), a liquid-impermeable anti leak sheet (22), and a liquid-holding absorbent (23) provided between the surface sheet (21) and the anti leak sheet, wherein the main part (2) is folded into a plurality of layers, and portions of the folded main part (2) that are overlapping with one another along opposite side edges of the main part (2) are bonded together.

2. An absorbent article according to claim 1, wherein a wing portion (3) extends from each side edge of the main part (2).

3. An absorbent article according to claim 2, wherein each wing portion (3) is folded onto the surface sheet (21) with an adhesive being applied on an outer surface thereof, therein defining an adhesive application area (3a), and a release liner is attached to the wing portions (3) so as to extend between the adhesive application areas (3a) of the wing portions (3).

4. An absorbent article according to claim 1 or 2, wherein an adhesive is applied to at least a portion of anti leak sheet (22), therein defining an adhesive application area (22a), with a release liner being attached to the adhesive application area (3a) of the anti leak sheet (22).

5. An absorbent article according to any one of claims 1-4, wherein the main part (2) is folded in two or more.

6. An absorbent article according to any one of claims 1-5, wherein the bonding is done by using at least one of thermal fusion bonding, ultrasonic fusion bonding and an adhesive.

7. An absorbent article according to claim 2 or 3, wherein bonding strength between the surface sheet (21) and the surface sheet (21) is set to be smaller than that between the wing portions (3) and the surface sheet (21).

8. An absorbent article according to claim 2 or 3, wherein bonding strength between the surface sheet (21) and the liquid-impermeable anti leak sheet (22) is set to be smaller than that between the wing portions (3) and the surface sheet (21).
